# EUROPEAN PATENT SPECIFICATION

(11) **EP 3 533 408 B1**
(45) Date of publication and mention of the grant of the patent: **14.06.2023**
(21) Application number: 18159216.3
(22) Date of filing: 28.02.2018
(51) Int. Cl.: A61B 34/20, A61B 90/00, A61B 34/10

(54) **METHOD, SYSTEM, COMPUTER PROGRAM PRODUCT AND COMPUTER-READABLE MEDIUM FOR TEMPORARILY MARKING A REGION OF INTEREST ON A PATIENT**
VERFAHREN, SYSTEM, COMPUTERPROGRAMMPRODUKT UND COMPUTERLESBARES MEDIUM ZUM TEMPORÄREN MARKIEREN EINES INTERESSIERENDEN BEREICHS AUF EINEM PATIENTEN
PROCÉDÉ, SYSTÈME, PRODUIT-PROGRAMME INFORMATIQUE ET SUPPORT LISIBLE PAR ORDINATEUR POUR MARQUER TEMPORAIREMENT UNE RÉGION D'INTÉRÊT SUR UN PATIENT

(43) Date of publication of application: 04.09.2019
(73) Proprietor: Siemens Healthcare GmbH, 91052 Erlangen (DE)
(72) Inventor: Schneider, Rainer, 91054 Erlangen (DE)

(56) References cited:
- WO-A2-2011/063266
- US-A1- 2009 175 406
- US-A1- 2015 366 628
- US-A1- 2016 367 169
- US-A1- 2017 042 622
- US-A1- 2017 165 028
- US-A1- 2017 312 035
- US-B1- 6 314 311

## Description

The present invention deals with a method, a system, a computer program product, and a computer-readable medium for temporarily marking a region of interest on a patient.

It is well known in the prior art to record medical imaging data sets by medical imaging devices such as magnetic resonance tomography (MRT) scanners. The recorded medical imaging data set can be reconstructed and can subsequently be presented, for example, on a screen for gaining insight to an inner structure of the patient, i.e., for visualizing organs and different types of tissues. The MRT scanner even allows observing or examining the inner structure of the patient during a treatment, such as a biopsy, by using an operation tool, such as a needle. However, a proper alignment of the operation tool relative to a body surface of the patient is difficult when the patient is located inside a bore of the medical imaging device for recording the medical imaging device. Therefore, the patient is typically transferred to a position outside of the bore. Afterwards, the operation tool is orientated relative to the patient and/or is inserted. In a subsequent step, the position of the operation tool is checked by transferring the patient back into the medical imaging device for recording another medical imaging data set that confirms the proper alignment of the operation tool. This procedure is repeated until the proper alignment is established. However, such a trial and error strategy for aligning the operation tool is time consuming and puts strain on the patient.

To ease the orientation of the operation tool, the state of the art knows of, for example, robots or inserters compatible with the medical imaging device. However, tactile feedback, handling, and hardware availability are quite limited.

Furthermore, robotics consist of many rigid structures which introduce an addition safety burden.

In an alternative approach, a planning software is used, wherein the planning software, for example, plans an entry point and the trajectories for the operation tool. Further, optical navigation devices have been installed to track the operation tool. However, the navigation or planning software relies on static image data which might become invalid as soon a movement of the patient occurs. Moreover, the systems must be re-calibrated as soon as the table position is moving.

For example, US 2017/165028 A1 discloses a projection system for image projection of anatomical features on a subject. Intraoperative position markers are disposed on a first body surface, and a moveable first detector with a detection line of sight is provided. A control unit is arranged for registering detected intraoperative position markers with a plurality of preoperative position markers in preoperative images.

In US 6, 314,311 B1 a registration system for use in connection with an image guided surgery system is disclosed. It includes a medical diagnostic imaging apparatus for collecting image data from a subject. An image data processor reconstructs an image representation of the subject from the image data. An image projector depicts the image representation on the subject. In a preferred embodiment, the image projector depicts the image representation on the subject such that registration between the subject and the image representation is readily apparent.

According to US 2016/367169 A1 a method for the selection of at least one item of examination information for a medical imaging examination before the medical imaging examination begins is provided, wherein position data of the patient are detected using a position detector. The detected position data are evaluated by a position data evaluation processor, with a patient image that shows a surface image of the patient being created on the basis of the detected position data.

The method of US 2017/042622 A1 suggests a surgical guidance and image registration, in which three-dimensional image data associated with an object or patient is registered to topological image data obtained using a surface topology imaging device. The surface topology imaging device may be rigidly attached to an optical position measurement system that also tracks fiducial markers on a movable instrument.

WO 2011/063266 A2 discloses an augmentation device for an imaging system which has a bracket structured to be attachable to an imaging component, and a projector attached to the bracket. The projector is arranged and configured to project an image onto a surface in conjunction with imaging by the imaging system.

A surgical navigation system according to US 2017/312035 A1 comprises a computer system, a tracking system and a projector. The computer system includes instructions for performing a surgical plan. The tracking system is electronically connected to the computer system and has a viewing area in which markers can be located within a three-dimensional space relative to the viewing area. The projector is electronically connected to the computer system and has a beam projectable into the viewing area to display a step of the surgical plan based on a location of a marker.

It is therefore an object of the present invention to provide a method that simplifies the alignment of an operation tool relative to the patient.

This object is achieved by the method according to claim 1 for temporarily marking a region of interest on a patient, by the system according to claim 10, the computer program product according to claim 11, and by the computer-readable computer medium according to claim 12.

Preferred embodiments are defined in the dependent claims.

According to a first aspect of the present invention, a method for temporarily marking a region of interest on a patient is provided, comprising
- providing a medical imaging device having a bore and a table, wherein the table is transferable between a first position, in which at least a part of the patient is located inside the bore, and a second positon, in which at least the part of the patient is located outside the bore, wherein a medical image data set of the patient, comprising the body surface of the patient, is recordable by the medical imaging device, when the patient is in the first position;
- providing an optical sensor device for recording an image of the patient for registering a position and/or a motion of the patient;
- providing a light source for marking a spot on the body surface of the patient as the region of interest in the second position, wherein for matching their coordinate systems, the medical imaging device and the optical sensor device are calibrated with each other permanently, in particular in an installation step,
- recording a medical image data set of the body surface of the patient by the medical imaging device,
- reconstructing and presenting the medical image data set on a screen to a user,
- receiving a region of interest at the surface of the patient selected by the user on the screen,
wherein after calibration, the medical imaging device and the light source share a common coordinate system and are correlated to each other permanently, and the method further comprises:
- recording an image data set of the body surface of the patient by the optical sensor device when the patient is in the second position,
- co-registering the medical image data set and the image data set, and
- steering or controlling the light source such that a spot on the body surface of the patient corresponding to the region of interest is marked, when the patient is in the second position.

As a consequence, the marked spot on the body surface of the patient can assist the user, i.e., an operator, with the orientation at the surface of the patient. Basis for identifying the proper spot in the second position of the table is the calibration of the medical imaging device and the optical sensor device such that the medical imaging device and the optical sensor device share a common coordinate system. Thus, it is possible to coordinate the light source, in particular a beam emitted by the light source, based on information extracted both from the medical imaging data set recorded by the medical imaging device and from an image data set recorded by the optical sensor device. Preferably the calibration is performed during the installation of the optical sensor device and/or the medical imaging device. It is also thinkable that the medical imaging device is upgraded by adding the optical sensor device and the light source for marking the body surface of the patient and that the calibration is performed in context of the upgrade.

The term "calibrated" means that the medical imagining device and the light source share a common coordinate system, after calibration, and are correlated to each other permanently, according to the present invention.

For example, the coordination is realized by a one-time calibration that matches the respective coordinate system of the medical imaging device and the optical sensor device. It is also thinkable that information regarding the position of the medical imaging device and the optical sensor device are exchanged permanently. A control unit is preferably used for coordination. In particular, the control unit comprises a processor being configured for
- coordinating the medical imaging device and the light source and/or
- steering the light source.

For example, the processor is part of a network or a workstation of the medical imaging device. A workstation might be a (personal) computer, a virtual running machine on host hardware, a microcontroller, or an integrated circuit. As an alternative, the workstation can be a real or a virtual group of computers. Preferably, the workstation comprises a calculation unit and a memory unit. A calculation unit can comprise hardware elements and software elements, for example, the processor, in particular a microprocessor, or a field programmable gate array. A memory unit can be embodied as a non-permanent main memory (e.g. random access memory) or a permanent mass storage (e.g. a hard disk, USB stick, SD card, solid state disk). It is also thinkable that at least one of the steps of the method is performed on a server or at the cloud.

Preferably, the medical imaging device is a magnetic resonance tomography (MRT) scanner. Advantageously, the MRT scanner needs no ionizing radiation and provides a superior soft tissue contrast. In particular, the table is located at least partially in the bore of the MRT scanner in the first position. It is provided that the medical imaging device records a medical imaging data set that is reconstructed and presented on a screen according to the present invention, such as a screen of a workstation, a smartphone, a tablet or the like. Thus, it is possible to identify a target area inside the patient body. According to the present invention, it is provided to use the visualized medical imaging data set for selecting a region of interest at the surface of the patient. The region of interest might be an entry point for a planned intervention.

The term "table" is generic for each device able to transfer the patient between the first position and the second position. The table is part of the medical imaging device. By using the optical sensor device, it is advantageously possible to take the current position of the patient into account. As a consequence, the accuracy of marking the proper region of interest on the surface of the patient can be considered in real time. The relative position of the optical sensor device to the medical imaging device is fixed. For example, the optical sensor device is fixed to a wall or ceiling. Thus, only one calibration is needed to match the corresponding coordinate systems. It is provided that the information about the position and/or the movement of the patient recorded by the optical sensor device is used for correcting the current position of the mark on the patient.

According to a preferred embodiment, it is provided that an information data set is transferred from the medical imaging device and/or from the optical sensor to the control unit for calibration, for example, during installation and/or before each operation cycle of the medical imaging device. The information might include information regarding the absolute position of the medical imaging device and the optical sensor device. It is further thinkable that the medical imaging device, the optical sensor device, and/or the light source exchange information such as a current position of the table and/or a status of the medical imaging device, and/or information regarding the medical imaging data set, in particular the visualized medical imaging data set. For example, it is also thinkable that specifications regarding the region of interest are transferred. Thereby, it is thinkable that the user analyses the visualized medical imaging data set and, for instance, selects a region of interest. Subsequently, the information about the region of interest is transferred to the control unit. Thus, it is advantageously possible to inform the control unit about the region of interest and preferably at least about a rough estimation of the position of the patient. As a consequence, the control unit steers or controls the light source such that the corresponding spot on the body of the patient is visually marked/highlighted according to the present invention.

According to the present invention, it is provided that a medical image data set is recorded by the medical imaging device and that an image data set of the body surface of the patient is recorded by the optical sensor device, wherein the medical image data set and the image data set are co-registered. Advantageously, the calibration of the medical imaging device and the optical sensor device simplifies the co-registering of the medical image data set and the image data set, which increases the accuracy for marking the proper spot on the body surface of the patient. By co-registration, the medical image data set and the image data set are transformed such that they have a common coordinate system.

Preferably, it is provided that the image data set of the body surface of the patient is recorded in the first position and/or the second positon. By observing the patient in the first position during recording the medical image data set and observing the patient in the second position by the optical sensor device, it is advantageously possible to identify movements of the patient and correct the light source by considering the movement of the patient. This increases the probability of marking the proper spot on the body surface of the patient. For registering a movement of the patient, the co-registered medical image data set and the image data set are used. For example, the surface of the body is observed both in the visualized medical image data set and in the visualized image data set. Due to the common coordinate system, it is possible to register a movement of the patient. In particular, it is provided that a difference between the body surfaces of the patient observed by the medical imaging device and the body surface of the patient observed by the optical sensor is determined, and the determined difference is compared to a threshold value. When the difference exceeds the threshold value, the position of the spot on the body surface of the patient is corrected.

Preferably, the optical sensor observes the movement continuously during the transfer between the first position and the second positon. Thus, it is advantageously possible to register a movement of the patient during the transfer. In other words: the optical sensor device permanently observes the patient. Therefore, the light source can correct the direction of the emitted beam at any time.

According to a preferred embodiment, it is provided that at least one image data set of the body surface of the patient is respectively recorded before and after recording the medical image data set. Thus, it is advantageously possible to register a movement of the patient between a time before recording the medical image data set and after recording the medical image data set. This comparison gives a rough estimation about the movement of the patient.

The light source preferably comprises a steerable laser and/or a projector. Thereby, a laser spot can be easily directed to the region of interest. Due to the relatively small spot size of a laser, it is advantageously possible to accurately project the laser on the respective spot on the surface of the patient.

Preferably, the light source is included in the optical sensor device. Thus, matching between the coordinate system of the optical sensor device and the coordinate system of the light source is not needed.

According to a preferred embodiment, it is provided that the optical sensor device is a device for range imaging, in particular a 3D camera or 4D camera. Preferably, the recording device is a device for range imaging, such as a 3D-camera and/or a 4D camera. Thus, it is possible to provide real-time 3D images of the patient on the table as an image data set. In general, the device for range imaging uses techniques that are used to produce a 2D image showing the distance to points in a scene from a specific point normally associated with some type of sensor device. Thereby, the resulting image has pixel values that correspond to the distance. Preferably, the pixel values can be given directly in physical units, such as meters. In particular, it is provided that the surface of the patient is deduced by the 3D camera. Thus, it is advantageously possible to, in addition to the current position of the patient, also consider the current position of the surface of the patient, i.e., the skin of the patient. In this way, the accuracy of highlighting the proper spot is further increased. Moreover, it is also thinkable to consider the physiological movements of the patient that are associated with a breathing cycle. The basis for this might be observing the patient with a 4D camera which also registers a time development.

In particular, it is provided that a motion of the patient and/or a breathing cycle are recorded by the optical sensor device. Based on the motion and/or the breathing cycle, it is advantageously possible to accurately determine the current position and/or orientation of the skin of the patient. In particular, observing the motion of the patient and the breathing cycle allows to correspondingly correct the position of the mark on the patient, in particular in real time. In other words: the control unit registers a movement of the patient, in particular of the skin of the patient, for example associated with the breathing cycle of the patient, and the mark follows the said movement by realigning the light source.

According to another embodiment of the present invention, it is provided that additional information is pictured on the surface of the patient. For example, an anatomic image and/or a planned trajectory is projected as additional information onto the patient. This additional information further assists the user with orientation at the surface of the patient relative to the inner structure of the patient, in particular when the table is in the second position.

Preferably, it is provided that the image data set is provided to the medical imaging device for adapting a recording parameter in the first position. As a consequence, it is advantageously possible to adapt the medical imaging device based on the image data set recorded by the optical sensor device in the second position. For example, parameters of the medical imaging data set are adapted based on the image data set. Thus, the medical imaging device can take into account a potential movement of the patient that occurred when the table was arranged in the second position.

In another embodiment, it is provided that an operation tool is provided, wherein a relative orientation of the operation tool is visualized based on the medical image data set and the image data set when the table is in the second position. As a consequence, it is advantageously possible to provide a simulation of a reconstructed medical imaging data set showing both the inner structure and the current position of the operation tool in real time without recording a medical imaging data set. Thus, the user is informed of the current position of the operation tool during its intervention. Thus, alignment of the operation tool is further improved. For example, the operation tool is a needle provided for a biopsy.

According to another aspect of the present invention, a system for carrying out the steps of the method according to the present invention is provided.

A further aspect of the present invention is a computer program product for carrying out the steps of the method according to the present invention when the computer program product is loaded into a memory of a programmable device.

A further aspect of the present invention is a computer-readable medium on which program elements are stored that can be read and executed by a computer unit in order to perform steps of the method according to the present invention when the program elements are executed by the computer unit.

In the drawings:
Fig. 1 schematically shows a system for highlighting a region of interest on a patient and
Fig.2 schematically shows a block diagram illustrating a method for highlighting a region of interest on a patient.

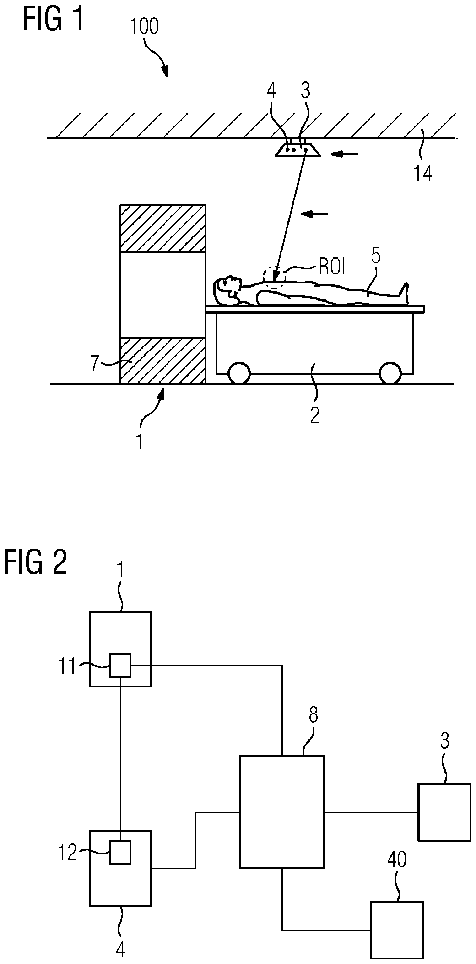

In figure 1, a system 100 for temporarily marking a region of interest ROI of a patient 5 according to a preferred embodiment of the present invention is shown. Such a system 100 comprises a medical imaging device 1, such as a magnetic resonance tomography (MRT) scanner. A medical imaging data set 11 is recorded by the medical imaging device 1, and subsequently, the medical imaging data set 11 is reconstructed for gaining an insight to the inner structure of the patient 5, i.e., organs, parts of organs, and their relative orientation or position. The MRT scanner shown in figure 1 further includes a bore 7. For recording the medical image data set, a patient 5 is placed inside the bore 7. The patient 5 is placed in the medical imagining device 1 by using a movable table 2 that can be transferred between a first position, i.e., a recording position, and a second position, i.e., a treatment or orientation position. In figure 1, the table 2 is arranged in the second position. The table 2 is arranged such in the first position that at least a part of the patient 5 is located inside the bore 7. Preferably, the part to be examined is located inside the bore 7 in the first position. In the second position, the table 2 is arranged such that the patient 5 is located at least partially outside of the medical imaging device 1. Preferably, the part that is to be examined is located outside the bore 7 in the second position of the table 2.

Preferably, an operation tool, such as a needle, is provided for performing a treatment, such as a biopsy, during recordal of the medical imaging data set 11. Preferably, the medical imaging device 1 is used for supporting the handling of the operation tool. For example, the medical imaging device 1, in particular the reconstructed medical imaging data set 11, helps to identify a target region, such as a tumor, inside the body of the patient and to identify a region of interest ROI at an outside of the patient 5. Such a region of interest ROI might be an entry point for entering the operation tool into the body of the patient. Additionally, it is thinkable to observe the position of the operation tool by the medical imaging device 1 during the treatment in order to ascertain the relative position of the operation tool during the treatment.

However, introducing the operation tool at the proper region of interest is quite difficult when the table 2 is arranged in the first position, since a user has to reach the region of interest, i.e., the entry point, from outside the bore 7 when the region of interest ROI might be located inside the bore 7. Therefore, the table 2 is transferred to the second position for placing and/or introducing the operation tool at the region of interest ROI, i.e., at the entry point. After introducing the operation tool, the table is transferred back to the first position for recording a medical imaging data set 11 that may confirm the proper alignment of the operation tool. This procedure is repeated until the proper alignment is established. As a result, the treatment of the patient takes a long time.

In order to increase the probability of a proper alignment of the operation tool, it is provided to mark a spot on a body surface of the patient 5 for highlighting the region of interest ROI in the second position. A light source 3 is used for marking the spot on the body surface of the patient 5. The light source 3 visually highlights or marks the region of interest ROI by directing the light source 3 on the region of the interest ROI. For example, the light source 3 comprises a laser for projecting a visual identification on the patient 5. Thus, the user is assisted in locating the proper region of interest ROI on the body surface of the patient 5 when the table 2 is in the second position.

For marking the proper spot on the surface of the patient 5, an optical sensor device 4, such as a camera, is further provided. Preferably, the optical sensor device 4 is configured for registering a current position and/or motion of the patient 5 arranged on the table 2. In particular, an image data set 12 of the patient 5 is recorded. For optimizing a co-registration of the medical image data set 11 and the image data set 12, it is provided that the medical imaging device 1 and the optical sensor device 4 are calibrated or coordinated, in order to match their coordinate systems, i.e. the coordinate system of the medical imaging device 1 and the coordinate system of the optical sensor device 4. The medical imaging device 1 and the light source 3 are calibrated such that they share a common coordinate system. For calibration, for example, an information data set is transferred between the medical imaging device 1 and the light source 3 and/or the optical sensor device 4 for exchanging information that helps to match their coordinate systems. For example, the information data set includes information about the relative position of the medical imaging device 1 and the optical sensor device.

After calibration, it is provided that a medical imaging data set 11 is recorded by the medical imaging device 1 and an image data set 12 of the body surface of the patient 5 is recorded by the optical sensor device 4, wherein the medical image data set 11 and the image data set 12 are co-registered. It is also thinkable that the user analyses a reconstructed medical imaging data set 11 and selects the region of interest ROI. Thus, it is possible to include information about the point of interest ROI into the medical image data set 11. Based on the medical image data set 11 and the image data set 12, the direction of a beam emitted by the light source 3 determined.

Thereby, it is provided that the optical sensor device 4 is arranged outside the bore 7. By using the optical sensor device 4, it is preferably possible to provide information about the position and/or motion of the patient 5 to a control unit 8 that, in turn, steers the light source 3. As a consequence the information about the current position and/or motion of the patient 5 and information extracted from the medical imaging data set 11 can be advantageously combined. Preferably, the optical sensor device 4 is a device for range imaging, such as a 3D-camera and/or a 4D camera. Thus, it is possible to provide real-time 3D images of the patient 5 on the table 2 as second information. In general, the device for range imaging uses techniques that are used to produce a 2D image showing the distance to points in a scene from a specific point. Thereby, the resulting image has pixel values that correspond to the distance. Preferably, the pixel values can be given directly in physical units, such as meters.

Preferably, the optical sensor device 4 is mounted to a ceiling 14 such that a field of view is directed to the table 2, for example, in the first position and/or the second position. Alternatively, it is thinkable that the optical sensor device 4 is mounted to a stative, wherein the optical sensor device 4 is arranged above the table 2. By using the device for range imaging, it is advantageously possible to record motions of the patient 5, determine a body surface of the patient 5, and track a breathing cycle and associated physiological movements of the patient 5. As a consequence, it is possible to take the current position and movement of the patient 5 into account for marking the spot on the surface of the patient 5. In particular, the position of the mark can be corrected based on the image data set 12 provided by the optical sensor device 4.

For example, the light source 3 is a steerable laser that is orientated such that the laser spots the entry point for a needle used for a biopsy when the table 2 is in the second position.

In addition, it is possible to use information extracted from the image data set 12 recorded by the optical sensor device 4 for simulating a visualisation of the position of the operation tool in the patient 5. For example, a recorded and visualized medical imaging data set 11 is provided to the control unit 8 together with the relative position of the operation tool and the body surface of the patient 5 extracted from the image data set 12 recorded by the optical sensor device 4. Preferably, the visualisation is presented on a screen 40. Based on the combination of the information extracted from the medical image data set 11 and the image data set 12 it is possible to simulate the position of the operation tool in the visualized medical imaging data set 11 and present another version of the visualized medical imaging data set 12 including the operation tool. As a consequence, it is possible to dynamically visualize the current position of the operation tool when the table is in the second position.

Furthermore, it is provided to use information extracted from the image data set 12 recorded by the optical sensor device 4 for adjusting the medical imaging device 1 when the table 2 returns into the first position. Thus, it is advantageously possible to take into account a potential movement of the patient 5 when the table 2 was in the second position, and adjust the parameters for recording the next medical imaging data set 11.

Preferably, it is provided that additional information and/or landmarks are pictured on the body of the patient 5, in particular next to the spot marked by the light source 3. The additional information might be a planned trajectory for the operation tool or an anatomical image that is projected on the body of the patient 4. This additional information or these landmarks can be used by the user for orientation.

## Claims

1. Method for temporarily marking a region of interest (ROI) at a body surface of a patient (5), comprising
- providing a medical imaging device (1) having a bore (7) and a table (2), wherein the table (2) is transferable between a first position, in which at least a part of the patient (5) is located inside the bore (7), and a second positon, in which at least the part of the patient (5) is located outside the bore (7), wherein a medical image data set (11) of the patient (5), comprising the body surface of the patient, is recordable by the medical imaging device (1), when the patient is in the first position;
- providing an optical sensor device (4) for recording an image of the patient (5) for registering a position and/or a motion of the patient (5);
- providing a light source (3) for marking a spot on the body surface of the patient (5) as the region of interest (ROI) in the second position,
wherein for matching their coordinate systems, the medical imaging device (1) and the optical sensor device (4) are calibrated with each other permanently, in particular in an installation step, wherein after calibration, the medical imaging device and the light source (3) share a common coordinate system and are correlated to each other permanently, the method further comprising
- recording a medical image data set (11) of the body surface of the patient (5) by the medical imaging device (1),
- reconstructing and presenting the medical image data set on a screen to a user,
- receiving a region of interest at the surface of the patient selected by the user on the screen,
- recording
an image data set (12) of the body surface of the patient (5) by the optical sensor device (4) when the patient is in the second position,
- co-registering the medical image data set (11) and the image data set (12) and
- steering or controlling the light source such that a spot on the body surface of the patient corresponding to the region of interest is marked, when the patient is in the second position.

2. Method according to claim 1, wherein the at least one image data set (12) of the body surface of the patient (5) is respectively recorded before and after recording the medical image data set (11).

3. Method according to one of the preceding claims, wherein the light source (3) comprises a steerable laser and/or a projector.

4. Method according to one of the claims 1 to 3, wherein the light source (3) is included in the optical sensor device (4) .

5. Method according to one of the preceding claims, wherein the optical sensor device (4) is a device for range imaging, in particular a 3D/4D camera.

6. Method according one of the preceding claims, wherein a motion of the patient (5) and/or a breathing cycle are recorded by the optical sensor device (4) and in particular the motion of the patient (5) and/or the breathing cycle are taken into account for marking the region of interest (ROI) on the body surface of the patient (5).

7. Method according to one of the preceding claims, wherein an additional information is pictured on the body of the patient (5), in particular next to the spot.

8. Method according to one of preceding claims, wherein the image data set (12) is provided to the medical imaging device (1) for adapting a recording parameter in the first position.

9. Method according to one of the preceding claims, wherein an operation tool is provided, wherein a relative orientation of the operation tool is visualized, in particular simulated, in a visualization of the medical image data set (11) based on the image data set (12) recorded by the optical sensor device.

10. System for temporarily marking a region of interest (ROI) at a body surface of a patient (5), comprising a medical imaging device (1) having a bore (7) and a table (2), wherein the table (2) is transferable between a first position, in which at least a part of the patient (5) is located inside the bore (7), and a second positon, in which at least the part of the patient (5) is located outside the bore (7), wherein a medical image data set (11) of the patient (5), comprising the body surface of the patient, is recordable by the medical imaging device (1), when the patient is in the first position;
an optical sensor device (4) for recording an image of the patient (5) for registering a position and/or a motion of the patient; and a light source for marking a spot on the body surface of the patient (5) as the region of interest (ROI) in the second position,
wherein for matching their coordinate systems, the medical imaging device (1) and the optical sensor device (4) are calibrated with each other permanently, in particular in an installation step, wherein after calibration, the medical imaging device and the light source (3) share a common coordinate system and are correlated to each other permanently,
wherein the system is configured for
- recording a medical image data set (11) of the body surface of the patient (5) by the medical imaging device (1),
- reconstructing and presenting the medical image data set on a screen to a user and
- receiving a region of interest at the surface of the patient selected by the user on the screen,
- recording an image data set (12) of the body surface of the patient (5) by the optical sensor device (4) when the patient is in the second position
- co-registering the medical image data set (11) and the image data set (12) position, and
- steering or controlling the light source such that a spot on the body surface of the patient corresponding to the region of interest is marked, when the patient is in the second position.

11. Computer program product for carrying out the steps of the method according one of the claims 1 to 9, when the computer program product is loaded into a memory of a programmable device connected to a system according to claim 10.

12. Computer-readable medium on which program elements are stored that can be read and executed by a computer unit in order to perform steps of the method according one of the claims 1 to 9, when the program elements are executed by the computer unit when the computer unit is connected to a system according to claim 10.

## Patentansprüche

1. Verfahren zum temporären Markieren einer Region von Interesse (Region of Interest, ROI) an einer Körperoberfläche eines Patienten (5), umfassend:
- Bereitstellen einer medizinischen Bildgebungsvorrichtung (1) mit einem Patiententunnel (7) und einem Tisch (2), wobei der Tisch (2) zwischen einer ersten Position, in der wenigstens ein Teil des Patienten (5) im Innern des Patiententunnels (7) angeordnet ist, und einer zweiten Position, in der wenigstens der Teil des Patienten (5) außerhalb des Patiententunnels (7) angeordnet ist, verstellbar ist, wobei ein medizinischer Bilddatensatz (11) des Patienten (5), umfassend die Körperoberfläche des Patienten, von der medizinischen Bildgebungsvorrichtung (1) aufgezeichnet werden kann, wenn sich der Patient in der ersten Position befindet;
- Bereitstellen einer optischen Sensorvorrichtung (4) zum Aufzeichnen eines Bildes des Patienten (5) zum Registrieren einer Position und/oder einer Bewegung des Patienten (5);
- Bereitstellen einer Lichtquelle (3) zum Markieren eines Punkts auf der Körperoberfläche des Patienten (5) als Region von Interesse (Region of Interest, ROI) in der zweiten Position, wobei zum Abgleichen ihrer Koordinatensysteme die medizinische Bildgebungsvorrichtung (1) und die optische Sensorvorrichtung (4) permanent miteinander kalibriert werden, insbesondere in einem Installationsschritt, wobei sich, nach der Kalibrierung, die medizinische Bildgebungsvorrichtung und die Lichtquelle (3) ein gemeinsames Koordinatensystem teilen und permanent miteinander korreliert sind, wobei das Verfahren ferner umfasst:
- Aufzeichnen eines medizinischen Bilddatensatzes (11) der Körperoberfläche des Patienten (5) durch die medizinische Bildgebungsvorrichtung (1),
- Rekonstruieren und Präsentieren des medizinischen Bilddatensatzes auf einem Bildschirm für einen Benutzer,
- Empfangen einer Region von Interesse an der Oberfläche des Patienten gemäß Auswahl durch den Benutzer auf dem Bildschirm,
- Aufzeichnen eines Bilddatensatzes (12) der Körperoberfläche des Patienten (5) durch die optische Sensorvorrichtung (4), wenn sich der Patient in der zweiten Position befindet,
- Gemeinsames Registrieren des medizinischen Bilddatensatzes (11) und des Bilddatensatzes (12) und
- Steuern oder Kontrollieren der Lichtquelle, sodass ein Punkt auf der Körperoberfläche des Patienten, welcher der Region von Interesse entspricht, markiert wird, wenn sich der Patient in der zweiten Position befindet.

2. Verfahren gemäß Anspruch 1, wobei der wenigstens eine Bilddatensatz (12) der Körperoberfläche des Patienten (5) jeweils vor und nach dem Aufzeichnen des medizinischen Bilddatensatzes (11) aufgezeichnet wird.

3. Verfahren gemäß einem der vorstehenden Ansprüche, wobei die Lichtquelle (3) einen steuerbaren Laser und/oder einen Projektor umfasst.

4. Verfahren gemäß einem der vorstehenden Ansprüche 1 bis 3, wobei die Lichtquelle (3) in der optischen Sensorvorrichtung (4) enthalten ist.

5. Verfahren gemäß einem der vorstehenden Ansprüche, wobei die optische Sensorvorrichtung (4) eine Vorrichtung für die Abstandsbildgebung (Range Imaging, RI), insbesondere eine 3D/4D-Kamera, ist.

6. Verfahren gemäß einem der vorstehenden Ansprüche, wobei eine Bewegung des Patienten (5) und/oder ein Atemzyklus von der optischen Sensorvorrichtung (4) aufgezeichnet werden und insbesondere die Bewegung des Patienten (5) und/oder der Atemzyklus beim Markieren der Region von Interesse (Region of Interest, ROI) auf der Körperoberfläche eines Patienten (5) berücksichtigt werden.

7. Verfahren gemäß einem der vorstehenden Ansprüche, wobei zusätzliche Informationen auf dem Körper des Patienten (5), insbesondere neben dem Punkt, abgebildet werden.

8. Verfahren gemäß einem der vorstehenden Ansprüche, wobei der Bilddatensatz (12) für die medizinische Bildgebungsvorrichtung (1) zum Anpassen eines Aufzeichnungsparameters in der ersten Position bereitgestellt wird.

9. Verfahren gemäß einem der vorstehenden Ansprüche, wobei ein Operationswerkzeug bereitgestellt wird, wobei, in einer Visualisierung des medizinischen Bilddatensatzes (11) basierend auf dem von der optischen Sensorvorrichtung aufgezeichneten Bilddatensatz (12), eine relative Ausrichtung des Operationswerkzeugs visualisiert, insbesondere simuliert wird.

10. System zum temporären Markieren einer Region von Interesse (Region of Interest, ROI) an einer Körperoberfläche eines Patienten (5), umfassend eine medizinische Bildgebungsvorrichtung (1) mit einem Patiententunnel (7) und einem Tisch (2), wobei der Tisch (2) zwischen einer ersten Position, in der wenigstens ein Teil des Patienten (5) im Innern des Patiententunnels (7) angeordnet ist, und einer zweiten Position, in der wenigstens der Teil des Patienten (5) außerhalb des Patiententunnels (7) angeordnet ist, verstellbar ist, wobei ein medizinischer Bilddatensatz (11) des Patienten (5), umfassend die Körperoberfläche des Patienten, von der medizinischen Bildgebungsvorrichtung (5) aufgezeichnet werden kann, wenn sich der Patient in der ersten Position befindet;
eine optische Sensorvorrichtung (4) zum Aufzeichnen eines Bildes des Patienten (5) zum Registrieren einer Position und/oder einer Bewegung des Patienten; und
eine Lichtquelle zum Markieren eines Punkts auf der Körperoberfläche des Patienten (5) als Region von Interesse (Region of Interest, ROI) in der zweiten Position,
wobei zum Abgleichen ihrer Koordinatensysteme die medizinische Bildgebungsvorrichtung (1) und die optische Sensorvorrichtung (4) permanent miteinander kalibriert werden, insbesondere in einem Installationsschritt, wobei sich, nach der Kalibrierung, die medizinische Bildgebungsvorrichtung und die Lichtquelle (3) ein gemeinsames Koordinatensystem teilen und permanent miteinander korreliert sind, wobei das System ausgelegt ist zum:
- Aufzeichnen eines medizinischen Bilddatensatzes (11) der Körperoberfläche des Patienten (5) durch die medizinische Bildgebungsvorrichtung (1),
- Rekonstruieren und Präsentieren des medizinischen Bilddatensatzes auf einem Bildschirm für einen Benutzer und
- Empfangen einer Region von Interesse an der Oberfläche des Patienten gemäß Auswahl durch den Benutzer auf dem Bildschirm,
- Aufzeichnen eines Bilddatensatzes (12) der Körperoberfläche des Patienten (5) durch die optische Sensorvorrichtung (4), wenn sich der Patient in der zweiten Position befindet
- Gemeinsames Registrieren des medizinischen Bilddatensatzes (11) und des Bilddatensatzes (12) und
Steuern oder Kontrollieren der Lichtquelle, sodass ein Punkt auf der Körperoberfläche des Patienten, welcher der Region von Interesse entspricht, markiert wird, wenn sich der Patient in der zweiten Position befindet.

11. Computerprogrammprodukt zum Ausführen der Schritte des Verfahrens gemäß einem der Ansprüche 1 bis 9, wenn das Computerprogrammprodukt in einen Speicher einer programmierbaren Vorrichtung geladen wird, die mit einem System gemäß Anspruch 10 verbunden ist.

12. Computerlesbares Medium, auf dem Programmelemente gespeichert sind, die von einer Computereinheit gelesen und ausgeführt werden können, um Schritte des Verfahrens gemäß einem der Ansprüche 1 bis 9 durchzuführen, wenn die Programmelemente von der Computereinheit ausgeführt werden, wenn die Computereinheit mit einem System gemäß Anspruch 10 verbunden ist.

## Revendications

1. Procédé de marquage temporaire d'une région (ROI) à laquelle on s'intéresse à la surface du corps d'un patient (5), comprenant :
- on se procure un dispositif (1) d'imagerie médicale ayant un trou (7) et une table (2), dans lequel la table (2) peut être transférée entre une première position, dans laquelle au moins une partie du patient (5) est disposée à l'intérieur du trou (7), et une deuxième position, dans laquelle au moins une partie du patient (5) est disposée à l'extérieur du trou (7), dans lequel un ensemble (11) de données d'image médicale du patient (5), comprenant la surface du corps du patient, peut être enregistré par le dispositif (1) d'imagerie médicale, lorsque le patient est dans la première position ;
- on se procure un dispositif (4) capteur optique pour enregistrer une image du patient (5), pour enregistrer une position et/ou un mouvement du patient (5) ;
- on se procure une source (3) de lumière pour marquer une tache sur la surface du corps du patient (5), comme la région (ROI) à laquelle on s'intéresse dans la deuxième position,
dans lequel, pour faire correspondre leurs systèmes de coordonnées, le dispositif (1) d'imagerie médicale et le dispositif (4) capteur optique sont étalonnés l'un par rapport à l'autre en permanence, en particulier dans un stade d'installation, dans lequel, après étalonnage, le dispositif d'imagerie médicale et la source (3) de lumière partagent un système de coordonnées commun et sont corrélés l'un à l'autre en permanence, le procédé comprenant en outre
- l'enregistrement d'un ensemble (11) de données d'image médicale de la surface du corps du patient (5) par le dispositif (1) d'imagerie médicale,
- la reconstruction et la présentation de l'ensemble de données d'image médicale sur un écran à un utilisateur,
- la réception d'une région à laquelle on s'intéresse à la surface du patient sélectionnée par l'utilisateur sur l'écran,
- l'enregistrement d'un ensemble (12) de données d'image de la surface du corps du patient (5) par le dispositif (4) capteur optique, lorsque le patient est dans la deuxième position,
- le co-enregistrement de l'ensemble (11) de données médicales et de l'ensemble (12) de données d'image, et
- la conduite ou la commande de la source de lumière, de manière à ce qu'une tache à la surface du corps du patient correspondant à la région à laquelle on s'intéresse soit marquée, lorsque le patient est dans la deuxième position.

2. Procédé suivant la revendication 1, dans lequel on enregistre le au moins un ensemble (12) de données d'image de la surface du corps du patient (5) respectivement avant et après avoir enregistré l'ensemble (11) de données d'image médicale.

3. Procédé suivant l'une des revendications précédentes, dans lequel la source (3) de lumière comprend un laser pouvant être dirigé et/ou un projecteur.

4. Procédé suivant l'une des revendications 1 à 3, dans lequel la source (3) de lumière est incluse dans le dispositif (4) capteur optique.

5. Procédé suivant l'une des revendications précédentes, dans lequel le dispositif (4) capteur optique est un dispositif pour une imagerie de plage, en particulier une caméra 3D/4D.

6. Procédé suivant l'une des revendications précédentes, dans lequel on enregistre le mouvement du patient (5) et/ou un cycle de respiration par le dispositif (4) capteur optique, et en particulier le mouvement du patient (5) et/ou le cycle de respiration, sont pris en compte pour marquer la région (ROI) à laquelle on s'intéresse à la surface du corps du patient (5).

7. Procédé suivant l'une des revendications précédentes, dans lequel une information supplémentaire est mise en image sur le corps du patient (5), en particulier à côté de la tache.

8. Procédé suivant l'une des revendications précédentes, dans lequel l'ensemble (12) de données d'image est fourni au dispositif (1) d'imagerie médicale pour adapter un paramètre d'enregistrement dans la première position.

9. Procédé suivant l'une des revendications précédentes, dans lequel il est prévu un outil d'opération, dans lequel on visualise une orientation relative de l'outil d'opération, en particulier en la simulant, dans une visualisation de l'ensemble (11) de données d'image médicale, sur la base de l'ensemble (12) de données d'image enregistré par le dispositif capteur optique.

10. Système de marquage temporaire d'une région (ROI) à laquelle on s'intéresse à une surface du corps d'un patient (5), comprenant un dispositif (1) d'imagerie médicale ayant un trou (7) et une table (2), dans lequel la table (2) peut être transférée entre une première position , dans laquelle au moins une partie du patient (5) est disposée à l'intérieur du trou (7), et une deuxième position, dans laquelle au moins une partie du patient (5) est disposée à l'extérieur du trou (7), dans lequel un ensemble (11) de données d'image médicale du patient (5), comprenant la surface du corps du patient, peut être enregistré par le dispositif (1) d'imagerie médicale, lorsque le patient est dans la première position ;
un dispositif (4) capteur optique pour enregistrer une image du patient (5) pour enregistrer une position et/ou un mouvement du patient, et
une source de lumière pour marquer une tache à la surface du corps du patient (5), alors que la région (ROI) à laquelle on s'intéresse est dans la deuxième position,
dans lequel, pour faire correspondre leurs systèmes de coordonnées, le dispositif (1) d'imagerie médicale et le dispositif (4) capteur optique sont étalonnés l'un par rapport à l'autre en permanence, en particulier dans un stade d'installation, dans lequel, après étalonnage, le dispositif d'imagerie médicale et la source (3) de lumière partagent un système de coordonnées commun et sont corrélés l'un à l'autre en permanence, dans lequel le système est configuré pour :
- l'enregistrement d'un ensemble (11) de données d'image médicale de la surface du corps du patient (5) par le dispositif (1) d'imagerie médicale,
- la reconstruction et la présentation de l'ensemble de données d'image médicale sur un écran à un utilisateur,
- la réception d'une région à laquelle on s'intéresse à la surface du patient sélectionnée par l'utilisateur sur l'écran,
- l'enregistrement d'un ensemble (12) de données d'image de la surface du corps du patient (5) par le dispositif (4) capteur optique, lorsque le patient est dans la deuxième position,
- le co-enregistrement de l'ensemble (11) de données médicales et de l'ensemble (12) de données d'image, et
- la conduite ou la commande de la source de lumière, de manière à ce qu'une tache à la surface du corps du patient correspondant à la région à laquelle on s'intéresse soit marquée, lorsque le patient est dans la deuxième position.

11. Produit de programme d'ordinateur pour effectuer les stades du procédé suivant l'une des revendications 1 à 9, lorsque le produit de programme d'ordinateur est chargé dans une mémoire d'un dispositif programmable relié à un système suivant la revendication 10.

12. Support, déchiffrable par ordinateur, sur lequel sont mis en mémoire des éléments de programme, qui peuvent être déchiffrés et exécutés par une unité informatique, afin d'effectuer les stades du procédé suivant l'une des revendications 1 à 9, lorsque les éléments de programme sont exécutés par l'unité informatique, lorsque l'unité informatique est reliée à un système suivant la revendication 10.
